# EUROPÄISCHE PATENTSCHRIFT

(11) **EP 1 352 918 B1**
(45) Veröffentlichungstag und Bekanntmachung des Hinweises auf die Patenterteilung: **26.07.2006**
(21) Anmeldenummer: 03006845.6
(22) Anmeldetag: 28.03.2003
(51) Int. Cl.: C08G 83/00, C08G 61/12, C07D 495/04, H01B 1/12

(54) **Alkylendioxythiophene und Poly(alkylendioxythiophen)e mit Urethangruppierungen enthaltenden Seitengruppen**
Alkylenedioxythiophenes and poly(alkylenedioxythiophene)s with side groups containing urethane groups
Alkylènedioxythiophènes et poly(alkylènedioxythiophène)s avec des groupes latérales contenant des groupements uréthane

(30) Priorität: 10.04.2002 DE 10215706
(43) Veröffentlichungstag der Anmeldung: 15.10.2003
(73) Patentinhaber: H.C. Starck GmbH, 38642 Goslar (DE)
(72) Erfinder: Reuter, Knud, Dr., 47800 Krefeld (DE)
(74) Vertreter: Feldhues, Michael L.F.

(56) Entgegenhaltungen:
- US-A- 5 111 327
- KROS A ET AL: "Poly(3,4-ethylenedioxythiophene)-based copolymers for biosensor applications" JOURNAL OF POLYMER SCIENCE: PART A: POLYMER CHEMISTRY, WILEY PERIODICALS, INC., Bd. 40, Nr. 6, 15. März 2002 (2002-03-15), Seiten 738-747, XP002244145
- WELSH D M ET AL: "EASILY FUNCTIONALIZED 3,4-ETHYLENEDIOXYTHIOPHENE" POLYMER PREPRINTS, AMERICAN CHEMICAL SOCIETY, US, Bd. 38, Nr. 2, 1997, Seite 320 XP001012694 ISSN: 0032-3934
- SCHOTTLAND P ET AL: "SYNTHESIS AND POLYMERIZATION OF NEW MONOMERS DERIVED FROM 3,4-ETHYLENEDIOXYTHIOPHENE" JOURNAL DE CHIMIE PHYSIQUE, SOCIETE DE CHIMIE PHYSIQUE, PARIS, FR, Bd. 95, Nr. 6, 1998, Seiten 1258-1261, XP001013494 ISSN: 0021-7689
- STEPHAN O ET AL: "NEW CATION-EXCHANGE MATERIAL BASED ON A SULFONATED 3,4-ETHYLENEDIOXYTHIOPHENE MONOMER" JOURNAL DE CHIMIE PHYSIQUE, SOCIETE DE CHIMIE PHYSIQUE, PARIS, FR, Bd. 95, Nr. 6, 1998, Seiten 1168-1171, XP000995727 ISSN: 0021-7689

## Beschreibung

Die Erfindung betrifft neue Alkylendioxythiophene mit Urethangruppierungen enthaltenden Seitengruppen, ihre Herstellung und deren oligo- und polymeren Derivate (Oligo- und Poly(alkylendioxythiophen)e).

Die Verbindungsklasse der π-konjugierten Polymere war in den letzten Jahrzehnten Gegenstand zahlreicher Veröffentlichungen. Sie werden auch als leitfähige Polymere oder als synthetische Metalle bezeichnet.

Wegen der erheblichen Delokalisierung der π-Elektronen entlang der Hauptkette zeigen diese Polymere interessante (nichtlineare) optische Eigenschaften, und nach Oxidation oder Reduktion stellen sie gute elektrische Leiter dar. Dadurch werden diese Verbindungen voraussichtlich eine führende und aktive Rolle auf verschiedenen praktischen Anwendungsgebieten übernehmen, wie z.B. in der Datenspeicherung, der optischen Signalverarbeitung, der Unterdrückung elektromagnetischer Störungen (EMI) und der Sonnenenergieumwandlung, sowie in wiederaufladbaren Batterien, lichtemittierenden Dioden, Feldeffekttransistoren, Leiterplatten, Sensoren und antistatischen Materialien.

Beispiele für bekannte π-konjugierte Polymere sind Polypyrrole, Polythiophene, Polyaniline, Polyacetylene, Polyphenylene und Poly(p-phenylen-vinylene).

Ein besonders wichtiges und technisch genutztes Polythiophen ist das Poly-3,4-(ethylen-1,2-dioxy)thiophen, das in seiner oxidierten Form sehr hohe Leitfähigkeiten aufweist und beispielsweise in der EP 339 340 A2 beschrieben ist. Eine Übersicht über zahlreiche Poly(alkylendioxythiophen)-Derivate, insbesondere Poly-3,4-(ethylen-1,2-dioxy)thiophen-Derivate, deren Monomerbausteine, Synthesen und Anwendungen gibt L. Groenendaal, F. Jonas, D. Freitag, H. Pielartzik & J. R.

Reynolds, Adv. Mater. 12 (2000) 481 - 494. US-A 5.111.327 beschreibt substituierte 3,4-Alkylendioxythiophene sowie deren leitfähige Polymerisationsprodukte.

Sehr hohe Leitfähigkeiten werden auch durch die Methodik der *in situ*-Polymerisation erzielt, bei der das monomere 3,4-(Ethylen-1,2-dioxy)thiophen mit Oxidationsmitteln, wie z. B. Eisen-III-tosylat, in Lösung zu einer hoch leitfähigen Schicht aus oxidiertem Poly-3,4-(ethylen-1,2-dioxy)thiophen umgesetzt wird. Diese Vorgehensweise wird z. B. für die Herstellung von Kondensatoren genutzt.

Trotz der guten Eigenschaften des Poly-3,4-(ethylen-1,2-dioxy)thiophens hinsichtlich Leitfähigkeit und Verarbeitbarkeit besteht Bedarf an weiteren Verbesserungen, insbesondere der Leitfähigkeit, ohne Beeinträchtigung oder sogar unter Verbesserung von Farbe und Transparenz, elektrochromen oder auch mechanischen Eigenschaften.

Diese Aufgabe wird überraschend durch die vorliegende Erfindung gelöst.

Gegenstand der Erfindung sind Verbindungen der Formel I, 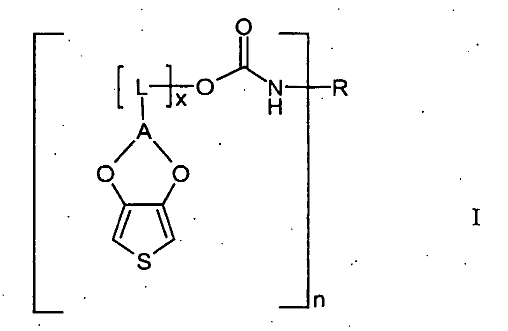 worin
- A: für einen-C₁-C₅-Alkylenrest steht, der an beliebiger Stelle gegebenenfalls über einen Linker L durch eine Urethangruppe substituiert ist und gegebenenfalls weitere Substituenten trägt,
- L: für eine Methylengruppe,
- x: für 0 oder eine ganze Zahl von 1 oder größer, bevorzugt für 0 oder eine ganze Zahl von 1 bis 6, besonders bevorzugt für 0 oder 1 steht,
- n: für eine ganze Zahl von 1 bis 4 steht und,
- R: für einen n-wertigen linearen oder verzweigten, gegebenenfalls substituierten, aliphatischen C₁-C₂₂-Rest, einen n-wertigen, gegebenenfalls substituierten, cycloaliphatischen C₃-C₁₂-Rest oder einen n-wertigen, gegebenenfalls substituierten, aromatischen C₆-C₁₄-Rest steht,
wobei für den Fall, dass n größer 1 ist, die Bedeutung von A und x unabhängig voneinander gleich oder verschieden sein kann.

Die Formulierung "wobei für den Fall, dass n größer 1 ist, die Bedeutung von A und x unabhängig voneinander gleich oder verschieden sein kann" ist im Sinne der Erfindung so zu verstehen, dass für den Fall, dass n größer 1 ist, jeweils die Bedeutung von A unabhängig voneinander gleich oder verschieden sein kann und die Bedeutung von x unabhängig voneinander gleich oder verschieden sein kann.
- A: steht bevorzugt für einen an beliebiger Stelle gegebenenfalls über einen Linker L durch eine Urethangruppe substituierten Methylen-, Ethylen- und Propylenrest, der gegebenenfalls weitere Substituenten, bevorzugt C₁-C₁₂₋Alkyl- oder Arylgruppen, trägt, wobei L die oben genannte Bedeutung hat.

Bevorzugt Gegenstand der Erfindung sind Verbindungen der Formel II, 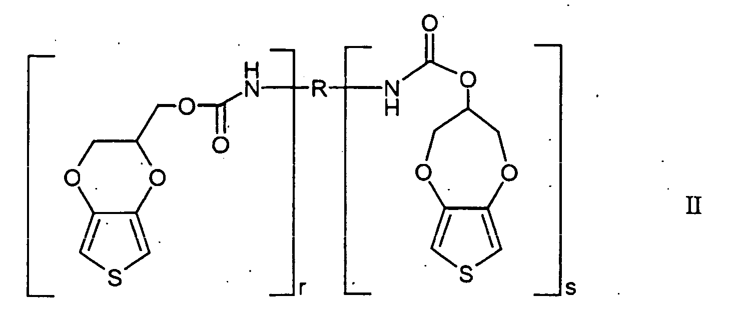 worin
- R: für einen n-wertigen linearen oder verzweigten, gegebenenfalls substituierten, aliphatischen C₂-C₂₂-Rest, einen n-wertigen, gegebenenfalls substituierten, cycloaliphatischen C₅-C₁₂-Rest, einen n-wertigen, gegebenenfalls substituierten, aromatischen C₆-C₁₄-Rest steht,
- r und s: unabhängig voneinander für ganze Zahlen von 0 bis 4 stehen, mit der Maßgabe, dass r + s = n ist und
- n: für eine ganze Zahl von 1 bis 4 steht.

Besonders bevorzugt sind dies Verbindungen der Formel II, worin R für einen linearen, gegebenenfalls substituierten C₆-C₁₂-Alkylenrest oder einen gegebenenfalls substituierten C₆-C₁₀-Cycloalkylenrest steht, r und s unabhängig voneinander für ganze Zahlen von 0 bis 2 stehen und n = r + s = 2 ist.

Besonders bevorzugt sind dies ebenfalls Verbindungen der Formeln II a und II b, entsprechend r = 0 oder s = 0 in Formel II, 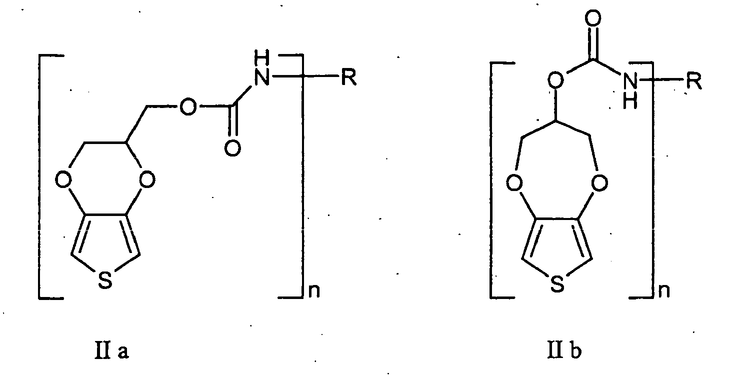 wobei
R und n die oben genannte Bedeutung haben.

Besonders bevorzugt sind dies ebenfalls Verbindungen der Formel III und IV, 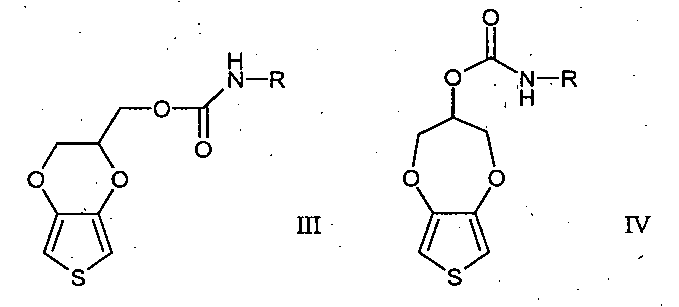 wobei
- R: für einen linearen oder verzweigten, gegebenenfalls substituierten C₁-C₂₂₋Alkylrest, einen gegebenenfalls substituierten C₃-C₁₂-Cycloalkylrest oder einen gegebenenfalls substituierten C₆-C₁₄-Arylrest steht, vorzugsweise für einen linearen C₁-C₁₂-Alkylrest oder einen C₆-C₁₀-Cycloakylrest, besonders bevorzugt für einen linearen C₄-C₁₀-Alkylrest.

Als Substituenten der oben und im Folgenden genannten Reste A und R kommen alle organischen Gruppen in Frage, die keine gegenüber Isocyanatgruppen aktiven H-Atome enthalten, beispielsweise Alkyl-, Cycloalkyl-, Aryl-, Halogen-, Ether-, Thioether-, Disulfid-, Sulfoxid-, Sulfon-, tertiäre Amino-, Aldehyd-, Keto-, Carbonsäureester-, Carbonat-, Cyano-, Alkylsilan- und Alkoxysilangruppen sowie Carboxylamidgruppen von sekundären Aminen.

Bevorzugt Gegenstand der Erfindung sind ebenfalls Mischungen aus Verbindungen der Formeln I bis IV, vorzugsweise Mischungen aus Verbindungen der Formel II, besonders bevorzugt Mischungen aus Verbindungen der Formel III und IV, die durch Einsatz von Mischungen der entsprechenden Ausgangsverbindungen V in dem im Folgenden beschriebenen ebenfalls erfindungsgemäßen Herstellungsverfahren erhältlich sind.

Gegenstand der Erfindung ist weiterhin ein Verfahren zur Herstellung der Verbindungen I bis IV, in dem Hydroxyverbindungen der Struktur V, 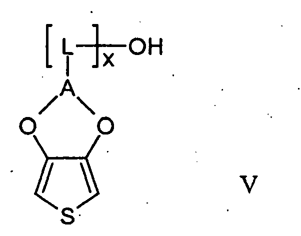 wobei
A, x und L die oben genannte Bedeutung haben, mit Monoisocyanaten, Di- oder höherfunktionellen Isocyanaten

R(NCO)ₙ,

wobei
R und n die oben genannte Bedeutung haben, umgesetzt werden.

Die Herstellung der neuen Verbindungen geschieht auf einfache Weise und in sehr guten Reinausbeuten durch Umsetzung von Verbindungen oder Mischungen aus Verbindungen der Formel V, besonders bevorzugt aus 2,3-Dihydrothieno[3,4-b]-[1,4]dioxin-2-ylmethanol (im Folgenden stets EDT-Methanol genannt) und/oder 3,4-Dihydro-2H-thieno[3,4-b][1,4]dioxepin-3-ol (im Folgenden stets Hydroxy-PDT genannt), mit Isocyanaten im etwa stöchiometrischen Verhältnis OH/NCO 1:1. Werden statt Monoisocyanaten Di- oder höherfunktionelle Isocyanate eingesetzt, so können die OH-funktionellen Thiophene auch im Unterschuss eingesetzt werden, und zwar bevorzugt so, dass nur eine NCO-Gruppe umgesetzt wird und alle weiteren NCO-Gruppen für Umsetzungen mit weiteren NCO-reaktiven Verbindungen genutzt werden können.

In den n-wertigen Isocyanaten R(NCO)ₙ (Mono-, Di- oder höherfunktionellen Isocyanaten) steht R für die vorangehend bereits mehrfach beschriebenen n-wertigen Reste.

Die besonders bevorzugten Ausgangsverbindungen EDT-Methanol = 2,3-Dihydrothieno[3,4-b][1,4]dioxin-2-ylmethanol und Hydroxy-PDT = 3,4-Dihydro-2H-thieno[3,4-b][1,4]dioxepin-3-ol sind leicht gemäß US-A 5.111.327 im Gemisch herzustellen. Bevorzugt werden für die Herstellung der erfindungsgemäßen Urethane solche Gemische eingesetzt, besonders bevorzugt Gemische mit Molverhältnissen EDT-Methanol/Hydroxy-PDT von 90:10 bis 65:35.

Es können aber auch die reinen Verbindungen eingesetzt werden, die z. B. durch eine chromatographische Trennung gemäß US-A 5.111.327 erhältlich sind. EDT-Methanol kann auch direkt in reiner Form gemäß Reynolds et al., Polym. Prepr. (Am. Chem. Soc., Div. Polym. Chem.) **38**(2), (1997), 320 mittels 2,3-Dibrompropylacetat hergestellt werden.

Die Hydroxyverbindungen der Formel V können allgemein in einer sauer katalysierten Umetherungsreaktion aus Alkantriolen und 3,4-Dialkoxythiophenen hergestellt werden. Geeignete 3,4-Dialkoxythiophene hierfür sind insbesondere diejenigen mit kurzkettigen n-Alkoxygruppen, bevorzugt Methoxy-, Ethoxy- und n-Propoxy-Gruppen. Diese Arbeitsweise ist prinzipiell in Adv. Mater. **11** (1999), S. 1379 - 1381 beschrieben. Beispielsweise sind durch den Einsatz geminaler 1,2-Diole mit einer zusätzlichen dritten Hydroxylgruppe, welche beispielhaft durch die folgende Formel VI beschrieben werden können,

HO-CH₂-CHOH-(L)ₓ-OH (VI)

wobei x und L die oben genannte Bedeutung haben, besonders bevorzugte Ausgangsverbindungen der Formel V zugänglich. Auch EDT-Methanol kann alternativ zu der in US-A 5.111.327 beschriebenen Herstellung durch Umetherung von 3,4-Dialkoxythiophenen mit Glycerin hergestellt werden. Weitere Beispiele sind die Herstellung der entsprechenden Verbindung mit x = 2 durch Umetherung von 3,4-Dialkoxythiophenen mit 1,2,4-Butantriol und der Verbindung mit x = 4 durch Umetherung von 3,4-Dialkoxythiophenen mit 1,2,6-Hexantriol. Als Nebenprodukte der Umetherungsreaktion mit 1,2,4-Butantriol können die ebenfalls erfindungsgemäßen Verbindungen der Formel V, 3,4-Dihydro-2H-thieno[3,4-b][1,4]dioxepin-2-yl-methanol und 2,3,4,5-Tetrahydrothieno[3,4-b][1,4]dioxocin-3-ol, erhalten werden. Die jeweils reinen Verbindungen können aus den gegebenenfalls erhaltenen Mischungen mittels chromatographischer Trennung isoliert werden.

Bis auf die bekannten Verbindungen der Formel V mit x = 0 oder 1, beispielsweise EDT-Methanol und Hydroxy-PDT, sind die Verbindungen der Formel V bisher in der Literatur nicht beschrieben und nach dem Verfahren gemäß US-A 5.111.327 auch nicht zugänglich. Überraschend konnten sie jedoch nach dem im vorangehenden Abschnitt beschriebenen Verfahren in einfacher Weise hergestellt werden.

Dies sind beispielsweise Hydroxyverbindungen der Formel V oder Mischungen aus Hydroxyverbindungen der Formel V, 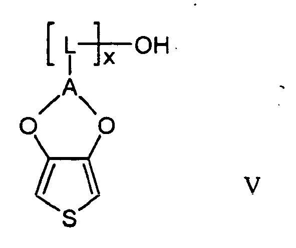 wobei
- A: für einen C₁-C₅-Alkylenrest, bevorzugt für einen C₂-C₃-Alkylenrest steht, der an beliebiger Stelle gegebenenfalls über einen Linker L durch eine Hydroxygruppe substituiert ist und gegebenenfalls weitere Substituenten trägt,
- L: für eine Methylengruppe und
- x: für eine ganze Zahl größer als 1, bevorzugt für eine ganze Zahl von 2 bis 6 steht.

Als Substituenten für A kommen die oben bereits genannten in Frage.

Weiterhin Gegenstand der Erfindung ist ein Verfahren zur Herstellung von Oligo- und Poly(alkylendioxythiophen)en mit Urethangruppierungen enthaltenden Seitengruppen, dadurch gekennzeichnet, dass Verbindungen der Formeln I bis polymerisiert werden. Diese können beispielsweise oxidativ oder elektrochemisch, bevorzugt jedoch oxidativ polymerisiert werden. Dabei können entweder jeweils eine Verbindung ausgewählt aus den Formeln I bis IV oder Mischungen daraus, bevorzugt Verbindungen oder Mischungen aus Verbindungen der Formel II, besonders bevorzugt Verbindungen oder Mischungen aus Verbindungen der Formel III und/oder IV, polymerisiert werden. Eine weitere Möglichkeit ist beispielsweise die Halogenierung der Monomeren der Formeln I bis IV zu den entsprechenden 2,5-Dichlor- oder 2,5-Dibrom(alkylendioxy)thiophenen und anschließende reduktive Polykondensation mit Ni(0)-Verbindungen, wie z. B. Ni-COD-Verbindungen.

Mit dem erfindungsgemäßen Verfahren sind sowohl Homopolymere als auch Copolymere sowie die entsprechenden Oligomere erhältlich

Unter Oligo- und Poly(alkylendioxythiophen)en werden alle Verbindungen verstanden, in denen mehr als eine Einheit der Verbindungen gemäß den Formeln I bis IV durch Polymerisation oder Polykondensation miteinander verknüpft sind, d.h. die einen Polymerisationsgrad von 2 besitzen. Der Übergang von den Oligo- zu den Poly(alkylendioxythiophen)en bezüglich des Polymerisationsgrades ist fließend.

Die Formulierung "Urethangruppierungen enthaltende Seitengruppen" ist so zu verstehen, dass in den Alkylendioxythiophen-Einheiten die Urethangruppe als Substituent an den Alkylenrest A an einer beliebigen Stelle entweder über einen Linker L oder ohne diesen Linker L in der Weise gebunden ist, wie es in Formel I dargestellt ist, wobei A und L hier die oben genannte Bedeutung haben.

Gegenstand der Erfindung sind weiterhin Oligo- und Poly(alkylendioxythiophen)e mit Urethangruppierungen enthaltenden Seitengruppen, die durch Polymerisation wenigstens einer der erfindungsgemäßen Verbindungen der Formeln I bis IV erhältlich sind. .

In einer bevorzugten Ausführungsform sind dies Oligo- und Poly(alkylendioxythiophen)e mit Urethangruppierungen enthaltenden Seitengruppen, die wiederkehrende Einheiten folgender Formel VII enthalten, 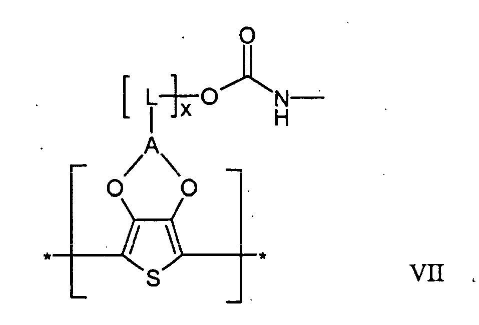 wobei
A, L und x die oben genannte Bedeutung haben, die innerhalb eines Polymers jeweils unabhängig voneinander gleich oder verschieden sein kann.

Beispielweise können dies, bezogen auf Verbindungen der Formel I für n = 1, Oligo- und Poly(alkylendioxythiophen)e der folgenden Formel VIII sein, 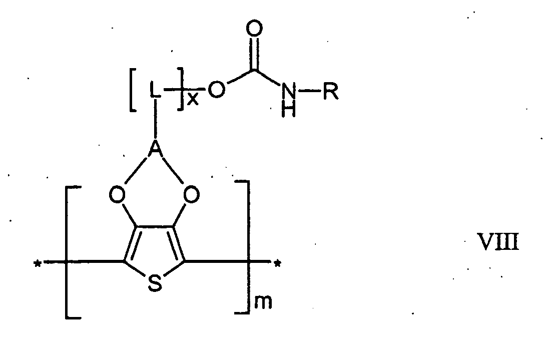 wobei
R, A, L und x die oben genannte Bedeutung haben, die innerhalb eines Polymers jeweils unabhängig voneinander gleich oder verschieden sein kann, und m mindestens 2 ist, bevorzugt mindestens 2 und maximal 2000, besonders bevorzugt mindestens 3 und maximal 50.

Bevorzugt Gegenstand der Erfindung sind Oligo- und Poly(alkylendioxythiophen)e mit Urethangruppierungen enthaltenden Seitengruppen, enthaltend wiederkehrende Einheiten der Formeln VI a und/oder VI b, 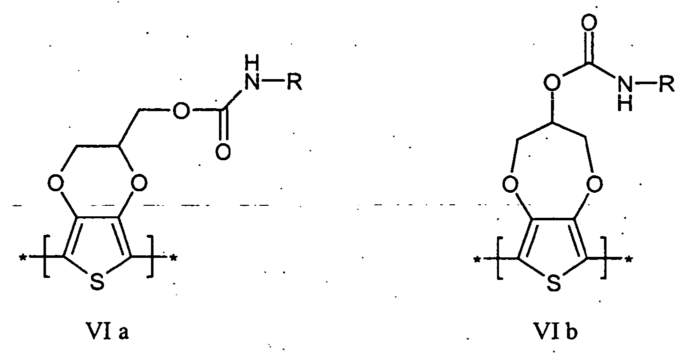 wobei
- R: für einen linearen oder verzweigten, gegebenenfalls substituierten C₁-C₂₂-Alkylrest, einen gegebenenfalls substituierten C₃-C₁₂-Cycloalkylrest oder einen gegebenenfalls substituierten C₆-C₁₄-Arylrest, vorzugsweise für einen linearen C₁-C₁₂-Alkylrest oder einen C₆-C₁₀-Cycloalkylrest, besonders bevorzugt für einen linearen C₄-C₁₀-Alkylrest, steht und die Zahl der wiederkehrenden Einheiten VI a und/oder VI b mindestens 2 ist.

Bevorzugt sind dies Oligo- und Poly(alkylendioxythiophen)e mit Urethangruppierungen enthaltenden Seitengruppen, wobei die Zahl der Einheiten der Formel VI a und die Zahl der Einheiten der Formel VI b unabhängig voneinander jeweils 0 bis 1000 ist, mit der Maßgabe dass die Summe der Zahl der wiederkehrenden Einheiten der Formeln VI a und/oder VI b im Oligo- und Poly(alkylendioxythiophen) mindestens 2 und höchstens 2000 ist.

Besonders bevorzugt sind dies Oligo- und Poly(alkylendioxythiophen)e mit Urethangruppierungen enthaltenden Seitengruppen, wobei die Zahl der Einheiten der Formel VI a 1 bis 50 ist und unabhängig davon die Zahl der Einheiten der Formel VI b 0 bis 30 ist, mit der Maßgabe dass die Summe der Zahl der wiederkehrenden Einheiten der Formeln VI a und/oder VI b im Oligo- und Poly(alkylendioxythiophen) mindestens 3 und höchstens 50 ist.

Dabei können die beiden unterschiedlichen Thiophenmonomeren blockartig, alternierend oder in einer ungeregelten, beliebigen Reihenfolge, bevorzugt aber in einer ungeregelten, beliebigen Reihenfolge im Oligo- und Polymeren angeordnet sein.

Ein Weg, der insbesondere zu den vorangehend beschriebenen Poly(alkylendioxythiophen)en in neutraler Form führt, ist die reduktive Polykondensation von 2,5-Dihalogen(alkylendioxy)thiophenen, die beispielsweise durch Halogenierung der Monomeren der Formeln I bis IV erhältlich sind. Durch oxidative Polymerisation der Verbindungen der Formeln I bis IV mit einem dem Fachmann bekannten Oxidationsmittel, wie beispielsweise Eisen-III-chlorid oder Eisen-III-tosylat, sind sowohl die neutralen als auch die kationischen Oligo- und Poly(alkylendioxythiophen)e erhältlich. Unter den Bedingungen der oxidativen Polymerisation, insbesondere bei Vorliegen eines Überschusses an Oxidationsmittel, kann daher bereits die Oxidation zum hoch leitfähigen Oligo- und Poly(alkylendioxythiophen)-Polykation erfolgen, muss jedoch nicht zwingend erfolgen. Alternativ kann die Darstellung der kationischen Oligo- und Poly(alkylendioxythiophen)e auch durch nachträgliche Oxidation der neutralen Poly(alkylendioxythiophen)e mithilfe dem Fachmann bekannter Oxidationsmittel erfolgen.

Somit sind ebenfalls Gegenstand der Erfindung Oligo- und Poly(alkylendioxythiophen)e mit Urethangruppierungen enthaltenden Seitengruppen hergestellt aus wenigstens einer Verbindung der Formeln I bis IV, dadurch gekennzeichnet, dass sie positive Ladungen tragen.

Die Zahl der positiven Ladungen kann mindestens 1 und maximal die Zahl der Alkylendioxythiophen-Einheiten sein.

Beispielweise können dies, bezogen auf Verbindungen der Formel I für n = 1, Oligo- und Poly(alkylendioxythiophen)e der folgenden Formel IX sein, 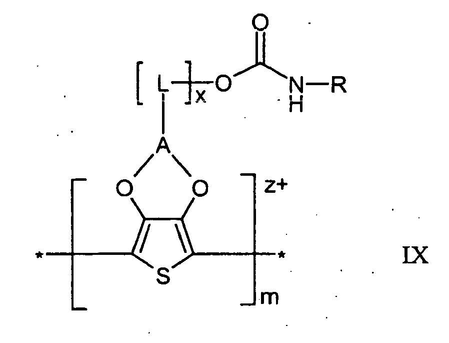 die z positive Ladungen tragen, wobei die Zahl z der positiven Ladungen mindestens 1 und maximal die Zahl der Wiederholungseinheiten m ist, vorzugsweise mindestens 1 und maximal m/2, besonders bevorzugt mindestens 1 und maximal m/3 bis m/4, und zur Neutralisation der z positiven Ladungen eine Zahl von z Gegenionen (Anionen) oder ein Oligo- oder Polyanion mit z negativen Ladungen, gegebenenfalls auch beide im Gemisch, in Gegenwart des Oligo- und Poly(alkylendioxythiophen)-Kations vorhanden ist. Oligo- oder Polyanionen können beispielsweise solche von oligo- und polymeren Carbon- oder Sulfonsäuren, wie z.B. Polyacrylsäuren oder Polystyrolsulfonsäuren sein. Die Oligo- und Poly(alkylendioxythiophen)-Kationen und Oligo- und Polyanionen können gemeinsam beispielsweise im Feststoff, in Lösung oder in Dispersion vorliegen.

Die durch Oxidation während oder nach der Polymerisation erzeugten positiven Ladungen der Polythiophen-Polykationen werden nicht in Formeln dargestellt, da ihre genaue Zahl und ihre Position nicht einwandfrei feststellbar sind.

Gegenstand der Erfindung sind außerdem zwei- oder dreidimensional vernetzte neutrale oder kationische Oligo- und Poly(alkylendioxythiophen)e, dadurch gekennzeichnet, dass sie aus wenigstens einer der Verbindungen der Formeln I bis IV durch oxidative Polymerisation erhältlich sind, vorzugsweise aus Verbindungen oder Mischungen von Verbindungen der Formel II, besonders bevorzugt aus Verbindungen der Formeln II a und/oder II b.

Überraschend zeigen die erfindungsgemäßen Oligo- und Poly(alkylendioxythiophen)e mit Urethangruppierungen enthaltenden Seitengruppen eine im Vergleich zu bekannten Verbindungen höhere Leitfähigkeit, ohne dabei an Transparenz zu verlieren. Aufgrund dieser vorteilhaften Eigenschaften eignen sie sich besonders gut für den Einsatz in elektronischen und elektrischen Bauteilen.

Weiterhin Gegenstand der Erfindung ist somit die Verwendung der erfindungsgemäßen Oligo- und Poly(alkylendioxythiophen)e als Bestandteil in elektrischen oder elektronischen Bauteilen.

Die erfindungsgemäßen Monomeren I - IV, die daraus hergestellten erfindungsgemäßen neutralen Oligo- und Polymeren sowie die ebenfalls erfindungsgemäßen kationischen Polymeren können zur Herstellung von organischen elektrischen oder elektronischen Bauteilen, z.B. zur Herstellung von Leuchtelementen, Photozellen oder organischen Transistoren, zur Ausrüstung von Kunststofffolien für die Verpackung elektronischer Bauteile und für Reinraumverpackungen, für die antistatische Ausrüstung von Kathodenstrahlröhren, für die antistatische Ausrüstung von photographischen Filmen, als transparente Heizung, als transparente Elektroden, als Leiterplatten oder für elektrisch einfärbbare Fensterscheiben dienen. Durch oxidative Polymerisation ist es zum Beispiel möglich, leitfähige Schichten auf nicht leitenden Substraten, wie Glas, Keramik, Kunststoff etc., zu erzeugen. In Kondensatoren können die so erzeugten Schichten die Rolle der Kathode übernehmen.

Dabei können die erfindungsgemäßen Verbindungen der Formeln I bis IV im Gemisch mit dem Oxidationsmittel aus organischen Lösungsmitteln, wie beispielsweise Alkoholen, Methylenchlorid, Chloroform, N-Methylpyrrolidon etc., durch Aufrakeln, spin coating, Gießen, Tränken, etc. auf die Substrate aufgebracht werden.

Die folgenden, nicht als Beschränkung aufzufassenden Beispiele zeigen, dass mit den erfindungsgemäßen Oligo- und Poly(alkylendioxythiophen)en mit Urethangruppierungen enthaltenden Seitengruppen im Vergleich zu bekannten Verbindungen deutlich verbesserte Leitfähigkeiten erzielt werden können.

### Beispiele

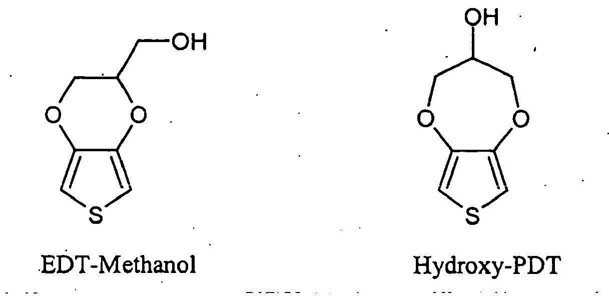

Das im Folgenden verwendete EDT-Methanol/Hydroxy-PDT-Gemisch 80:20 wurde gemäß US-A 5.111.327 hergestellt.

### Beispiel 1

13,0 g EDT-Methanol/Hydroxy-PDT-Gemisch 80:20 wurde unter N₂ in 20 ml absolutiertem (abs.) Toluol gelöst. 7,48 g n-Butylisocyanat wurden unter Rühren rasch bei Raumtemperatur (23°C) zugetropft. Danach wurde das Reaktionsgemisch 2 h auf 100°C erhitzt. Danach war die Umsetzung laut Dünnschicht-Chromatographie (Laufinittel Chloroform/Methanol 10:1) vollständig. Nach Abkühlen auf Raumtemperatur wurde einige min mit 20 ml Methanol verrührt; anschließend wurden alle Lösemittel bei 20°C/20 mbar abgezogen. Der ölige Rückstand wurde mit CHCl₃/Ethylacetat 1:1 als Laufmittel über eine Kieselgelsäule gereinigt. Man erhielt 3,3 g reines sowie weitere 14,4 g leicht verunreinigtes Produkt, Gesamtausbeute: 86,4 % d. Th. Für weitere Untersuchungen und Umsetzungen wurde stets die reine Fraktion verwendet.

### Beispiel 2

12,0 g EDT-Methanol/Hydroxy-PDT-Gemisch 80:20 wurde unter N₂ in 20 ml abs. Toluol gelöst. 8,86 g n-Hexylisocyanat wurden unter Rühren rasch bei Raumtemperatur (23°C) zugetropft. Danach wurde das Reaktionsgemisch 11,5 h auf 100°C erhitzt. Danach war die Umsetzung laut Dünnschicht-Chromatographie (Laufmittel Toluol/Ethylacetat 1:1) vollständig. Nach Abkühlen auf Raumtemperatur wurde einige min mit 25 ml Methanol verrührt; anschließend wurden alle Lösemittel bei 20 mbar abdestilliert. Der ölige Rückstand wurde mit Toluol/Ethylacetat 1:1 als Laufmittel über eine Kieselgelsäule gereinigt. Man erhielt 14,27 g nach ¹H-NMR sauberes Produkt, Ausbeute: 68,4 % d. Th.

### Beispiel 3

12,0 g EDT-Methanol/Hydroxy-PDT-Gemisch 80:20 wurde unter N₂ in 20 ml abs. Toluol gelöst. 10,82 g n-Octylisocyanat wurden unter Rühren rasch bei Raumtemperatur (23°C) zugetropft. Danach wurde das Reaktionsgemisch 7,5 h auf 100°C erhitzt. Danach war die Umsetzung laut Dünnschicht-Chromatographie (Laufinittel Hexan/Toluol 1:1) vollständig. Nach Abkühlen auf Raumtemperatur wurde einige min mit 25 ml Methanol verrührt; anschließend wurden alle Lösemittel bei 20 mbar abdestilliert. Rückstand: 20,0 g öliges Produkt, Ausbeute: 87,6 % d. Th.

### Beispiel 4

10,0 g EDT-Methanol/Hydroxy-PDT-Gemisch 80:20 wurde unter N₂ in 20 ml abs. Toluol gelöst. 12,72 g n-Dodecylisocyanat wurden unter Rühren in 30 min bei Raumtemperatur (23°C) zugetropft. Danach wurde das Reaktionsgemisch in 7 h auf 70°C erhitzt und weiter 16 h auf 80°C gehalten. Nach Abkühlen auf Raumtemperatur wurde das auskristallisierte Produkt aus 30 ml Methanol umkristallisiert und mit 10 ml Methanol nachgewaschen. Ausbeute 19,0 g = 85,3 % d. Th.; laut ¹H-NMR 78% Dodecylurethan des EDT-Methanols und 22 % Dodecylurethan des Hydroxy-PDT.

### Beispiel 5

8,0 g EDT-Methanol/Hydroxy-PDT-Gemisch 80:20 wurde unter N₂ in 20 ml abs. Toluol gelöst. 13,73 g n-Octadecylisocyanat wurden unter Rühren in 30 min bei Raumtemperatur (23°C) zugetropft. Danach wurde das Reaktionsgemisch 7,5 h bei 100°C gerührt. Danach war die Umsetzung nach Dünnschicht-Chromatographie (Laufmittel Toluol/Hexan 1:1) vollständig. Nach Abkühlen auf Raumtemperatur wurde das auskristallisierte Produkt mit 25 ml Methanol verrührt; alle Lösemittel wurden anschließend bei 20°C/20 mbar abgezogen und der Rückstand aus Methanol umkristallisiert. Ausbeute 16,15 g = 74,3 % d. Th..

### Beispiel 6

12,0 g EDT-Methanol/Hydroxy-PDT-Gemisch 80:20 wurde unter N₂ in 20 ml abs. Toluol gelöst. 8,72 g Cyclohexylisocyanat wurden unter Rühren rasch bei Raumtemperatur (23°C) zugetropft. Danach wurde das Reaktionsgemisch 2 h bei 100°C gerührt. Danach war die Umsetzung nach Dünnschicht-Chromatographie (Laufmittel Chloroform/Methanol 10:1) vollständig. Nach Abkühlen auf Raumtemperatur wurde das Gemisch mit 20 ml Methanol verrührt; alle Lösemittel wurden anschließend bei 20°C/20 mbar abgezogen. Der Rückstand wurde mit Petroleumbenzin verrieben und das feste Produkt abgesaugt. Ausbeute 17,5 g = 84,5 % d. Th..

### Beispiel 7

12,0 g EDT-Methanol/Hydroxy-PDT-Gemisch 80:20 wurde unter N₂ in 20 ml abs. Toluol gelöst. 8,3 g Phenylisocyanat wurden unter Rühren rasch bei Raumtemperatur (23°C) zugetropft. Danach wurde das Reaktionsgemisch 3,5 h bei ca. 100°C gerührt. Danach war die Umsetzung nach Dünnschicht-Chromatographie (Laufmittel Chloroform/Methanol 10:1) vollständig. Nach Abkühlen auf Raumtemperatur wurde das teilweise kristallisierte Gemisch mit 25 ml Methanol verrührt und der Feststoff abgesaugt. Ausbeute = 2,3 g Produkt (weiße Kristalle). Aus der Mutterlauge wurden durch Fällen mit Methanol weitere 1,5 g festes Produkt isoliert. Die danach verbliebene Mutterlauge wurde eingedampft und mit Toluol als Laufmittel über eine Kieselgelsäule aufgereinigt. Gesamtausbeute quantitativ.

### Beispiel 8

13,0 g EDT-Methanol/Hydroxy-PDT-Gemisch 80:20 wurde unter N₂ in 20 ml abs. Toluol gelöst. 7,48 g tert-Butylisocyanat wurden unter Rühren rasch bei Raumtemperatur (23°C) zugetropft. Danach wurde das Reaktionsgemisch 7,5 h auf ca. 100°C erhitzt. Nach Abkühlen auf Raumtemperatur wurde einige min mit 25 ml Methanol verrührt; anschließend wurden alle Lösemittel bei 20°C/20 mbar abgezogen. Der ölige Rückstand wurde mit CHCl₃/Ethylacetat 1:1 als Laufmittel über eine Kieselgelsäule gereinigt. Man erhielt 1,51 g = 7,4 % d. Th. Produkt als hellgelbes Öl. Nach ¹H-NMR 93 % des tert-Butylurethans des EDT-Methanol und 7 % des tert-Butylurethans des Hydroxy-PDT.

### Beispiel 9

8,0 g EDT-Methanol/Hydroxy-PDT-Gemisch 80:20 wurde unter N₂ in 20 ml abs Toluol gelöst. 5,16 g Isophorondiisocyanat wurden unter Rühren rasch bei Raumtemperatur (23°C) zugetropft (NCO/OH = 1:1). Danach wurde das Reaktionsgemisch 5,5 h auf 100°C erhitzt. Nach Abkühlen auf Raumtemperatur wurde einige min mit 25 ml Methanol verrührt; anschließend wurden alle Lösemittel bei 20°C/20 mbar abgezogen. Ausbeute quantitativ.

### Beispiel 10

16,0 g EDT-Methanol/Hydroxy-PDT-Gemisch 80:20 wurde unter N₂ in 20 ml abs. Toluol gelöst. 7,8 g Hexamethylendiisocyanat wurden unter Rühren rasch bei Raumtemperatur (23°C) zugetropft (NCO/OH = 1:1). Danach wurde das Reaktionsgemisch 7,5 h auf ca. 100°C erhitzt. Nach Abkühlen auf Raumtemperatur wurde einige min mit 25 ml Methanol verrührt. Dabei fielen 5,1 g = 21,4 % d. Th. Produkt als farblose Kristalle aus.

### Beispiel 11

6,5 g EDT-Methanol/Hydroxy-PDT-Gemisch 80:20 wurde unter N₂ in 10 ml abs. Toluol gelöst. 4,87 g Ethyl-isocyanatoacetat wurden unter Rühren rasch bei Raumtemperatur (23°C) zugetropft. Danach wurde das Reaktionsgemisch 32 h auf 100°C erhitzt. Danach war die Umsetzung laut Dünnschicht-Chromatographie (Laufmittel Toluol/Ethylacetat 1:1) vollständig. Nach Abkühlen auf Raumtemperatur wurde einige min mit 20 ml Methanol verrührt; anschließend wurden alle Lösemittel bei 20 mbar abdestilliert. Der ölige Rückstand wurde mit Toluol/Ethylacetat 1:1 als Laufmittel über eine Kieselgelsäule gereinigt. Man erhielt 10,95 g nach ¹H-NMR sauberes Produkt als orangebraune, viskose Flüssigkeit, Ausbeute: 96,3 % d. Th.

### Beispiel 12

5,0 g EDT-Methanol/Hydroxy-PDT-Gemisch 80:20 wurde unter N₂ in 10 ml abs. Toluol gelöst. 7,18 g 3-(Triethoxysilyl)propylisocyanat wurden unter Rühren rasch bei Raumtemperatur (23°C) zugetropft. Dann wurde das Reaktionsgemisch 19 h auf 100°C erhitzt. Danach war die Umsetzung laut Dünnschicht-Chromatographie (Laufmittel Toluol/ Ethylacetat 1:1) vollständig. Nach Abkühlen auf Raumtemperatur wurde einige min mit 20 ml Ethanol verrührt; anschließend wurden alle Lösemittel bei 20 mbar abdestilliert. Rückstand: 11,86 g öliges, orangefarbenes, nach ¹H-NMR-Spektrum sauberes Produkt, Ausbeute: 97,4 % d. Th.

### Beispiel 13

Nach folgender Rezeptur wurden aus erfindungsgemäßen Verbindungen sowie Vergleichsverbindungen gemäß US-A 5.111.327 sowie 3,4-Ethylendioxythiophen kationische Polymere als Beschichtungen auf Glas hergestellt:

14 mmol der Thiophen-Verbindung werden mit 50 g einer 40%igen Lösung von Fe-III-tosylat in n-Butanol und 212 g n-Butanol gemischt. Die Lösung wird mit einem Rakel mit 60 µm Nassfilmdicke auf Glas aufgetragen und bei der angegebenen Temperatur die angegebene Zeit getrocknet. Anschließend wird der leitfähige Polymerfilm mit entionisiertem Wasser gewaschen. Die Oberflächenwiderstände werden nach Trocknen und gegebenenfalls Abkühlen auf 23°C mit der Zweipunkt-Methode mit Hilfe von Leitsilber-Kontakten gemessen. Die Polymerisation beginnt bereits langsam beim Mischen und ist nach der genannten Trocknungszeit beendet.

| **Thiophen-Verbindung** | **Trocknungs-Temperatur** | **Trocknungs-Zeit** | **Oberflächen-Widerstand (bei 23°C)** | |
|---|---|---|---|---|
| Beispiel 1 | 40°C | 10 min | 25 Ohm/sq | (erfindungsgemäß) |
| Beispiel 2 | 40°C | 10 min | 33 Ohm/sq | (erfindungsgemäß) |
| Beispiel 3 | 23°C | 20 min | 23 Ohm/sq | (erfindungsgemäß) |
| Beispiel 9 | 40°C | 10 min | 26 Ohm/sq | (erfindungsgemäß) |
| EDT-Methanol/Hydroxy-PDT 8:2 | 40°C | 10 min | 43 Ohm/sq | (Vergleich) |
| Bsp. 7 aus US-A 5.111.327 | 40°C | 10 min | 70 Ohm/sq | (Vergleich) |
| 3,4-Ethylendioxythiophen | 40°C | 10 min | 40 Ohm/sq | (Vergleich) |

Die Ergebnisse belegen, dass die erfindungsgemäßen Verbindungen niedrigere Oberflächenwiderstände und somit höhere Leitfähigkeiten zeigen als die vermessenen bekannten Vergleichsverbindungen.

### Beispiel 14

4,532 g Eisen-III-chlorid werden in 100 ml Chloroform vorgelegt. 3,293 g des in Beispiel 2 hergestellten Urethans werden zudosiert und das Gemisch 16 h bei Raumtemperatur (23°C) gerührt. Anschließend wird das Reaktionsgemisch in eine Mischung aus 100 ml Methylenchlorid und 50 ml 26 %igem Ammoniak gefällt. Die Ammoniak-Phase wird abgetrennt und durch frische ersetzt. Dieser Vorgang wird zweimal wiederholt. Danach wird die organische Phase abgetrennt und mit 0,05-molarer wässriger EDTA-Lsg. ausgeschüttelt. Die org. Phase wird danach dreimal mit Wasser gewaschen und anschließend über Na₂SO₄ getrocknet. Nach Einengen im Wasserstrahlvakuum auf die Hälfte wird durch Eingießen in eine Mischung aus 220 ml Methanol und 33 ml 26 %igem Ammoniak das Produkt ausgefällt. Der abgesaugte Feststoff wird 1,4-%ig in Tetrahydrofuran gelöst (tiefrotviolette Lsg.) und danach 2 h mit 10 g eines sauren Ionenaustauschers auf Styrol/Divinylbenzol-Basis in der protonierten Form entionisiert.

Es handelt sich um das neutrale Polymer des Thiophenderivats aus Beispiel 2.

### Beispiel 15

4,116 g Eisen-III-chlorid werden in 100 ml Chloroform vorgelegt. 3,27 g des in Beispiel 3 hergestellten Urethans werden zudosiert und das Gemisch 16 h bei Raumtemperatur (23°C) gerührt. Anschließend wird das Reaktionsgemisch in eine Mischung aus 100 ml Methylenchlorid und 50 ml 26 %igem Ammoniak gefällt. Die Ammoniak-Phase wird abgetrennt und durch frische ersetzt. Dieser Vorgang wird zweimal wiederholt. Danach wird die organische Phase abgetrennt und mit 0,05-molarer wässriger EDTA-Lsg. ausgeschüttelt. Die org. Phase wird danach dreimal mit Wasser gewaschen und anschließend über Na₂SO₄ getrocknet. Nach Einengen im Wasserstrahlvakuum auf die Hälfte wird durch Eingießen in eine Mischung aus 220 ml Methanol und 33 ml 26 %igem Ammoniak das Produkt ausgefällt. Der abgesaugte Feststoff (0,7 g) wird 2,16-%ig in Tetrahydrofran gelöst (Tiefrotviolette Lsg.) und danach 2 h mit 10 g eines sauren Ionenaustauschers auf Styrol/Divinylbenzol-Basis in der protonierten Form entionisiert.

Es handelt sich um das neutrale Polymer des Thiophenderivats aus Beispiel 3.

Nach GPC (Polystyrol-Eichung, RI (Brechungsindex)-Detektion) liegt das Molekulargewicht (Gewichtsmittel) bei M_{w} = 12.200.

Die Beispiele 14 und 15 belegen hier die vorangehend bereits beschriebene Möglichkeit der gezielten Darstellung der neutralen Polymere auf dem Weg der oxidativen Polymerisation.

### Allgemeine Vorschrift zur Synthese der Hydroxyverbindungen der allgemeinen Formel V

Beispielhaft kann die Synthese der Hydroxyverbindungen der allgemeinen Formel V durch Umetherung so erfolgen, dass 3,4-Di-n-propoxythiophen und ein Alkantriol, bevorzugt das Alkantriol im Überschuss, mit p-Toluolsulfonsäure als Katalysator unter N₂ für 2 h (oder länger) erhitzt werden, wobei n-Propanol langsam abdestilliert wird. Nach Abkühlen wird die verbleibende Flüssigkeit mit Methylenchlorid verdünnt, mit Wasser neutral gewaschen und die organische Phase über Na₂SO₄ getrocknet. Nach Entfernen des Lösungsmittels erhält man die Hydroxyverbindung der allgemeinen Formel V.

### Beispiel 16: Herstellung von 2-(2,3-Dihydrothieno[3,4-b][1,4]dioxin-2-yl)ethanol

9,06 g (45,2 mmol) 3,4-Di-n-propoxythiophen, 24,0 g (226 mmol) 1,2,4-Butantriol und 0,09 g (0,5 mmol) p-Toluolsulfonsäure wurden unter N₂ für 2 h auf 150 bis 160 °C (Badtemperatur) erhitzt. Während dieser Zeit wurden 5,89 g Destillat (im Wesentlichen n-Propanol) aufgefangen. Die abgekühlte Flüssigkeit wurde mit 50 ml Methylenchlorid verdünnt, mit Wasser neutral gewaschen und die organische Phase über Na₂SO₄ getrocknet. Danach wurde nach Filtrieren und Abdampfen des Methylenchlorids bei 1 mbar destilliert. Die zwischen 45 und 128 °C aufgefangene Fraktion (1,63 g = 19, 4 % d. Th.Gesamtausbeute an Umetherungsprodukten) bestand nach ¹H-NMR-Spektroskopie aus ca. 81,5 % 2-(2,3-Dihydrothieno[3,4-b][1,4]dioxin-2-yl)ethanol, 6,5 % 3,4-Dihydro-2H-thieno[3,4-b][1,4]dioxepin-2-ylmethanol und 12 % 2,3,4,5-Tetrahydrothieno[3,4-b][1,4]dioxocin-3-ol.

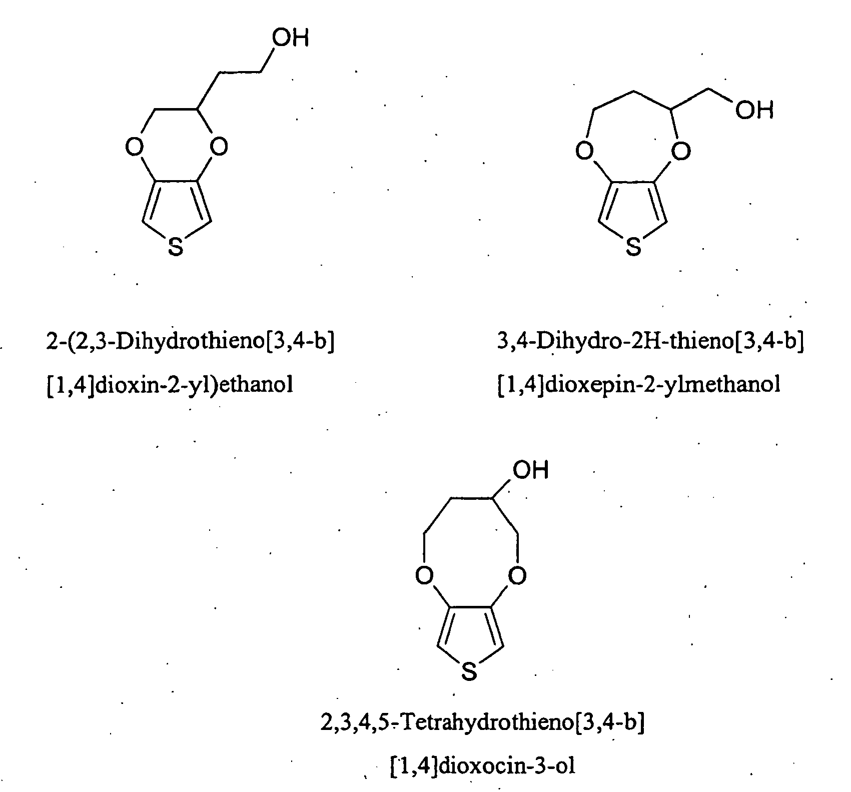

### Beispiel 17: Herstellung von 4-(2,3-Dihydrothieno[3,4-b][1,4]dioxin-2-yl)-1-butanol

9,06 g (45,2 mmol) 3,4-Di-n-propoxythiophen, 30,3 g (226 mmol) 1,2,6-Hexantriol und 0,09 g (0,5 mmol) p-Toluolsulfonsäure wurden unter N₂ für 3 h auf 150 bis 165°C (Badtemperatur) erhitzt. Während dieser Zeit wurden 5,8 g Destillat (im Wesentlichen n-Propanol) aufgefangen. Die abgekühlte Flüssigkeit wurde mit 50 ml Methylenchlorid verdünnt, mit Wasser neutral und Hexantriol-frei gewaschen und die organische Phase über Na₂SO₄ getrocknet. Nach Filtrieren und Abdampfen des Methylenchlorids wurde der Rückstand (6,73 g = 74,8 % d. Th.) durch ¹H-NMR-Spektroskopie (in CDCl₃ gegen TMS) über δ = 6,30 ppm (2 H, Thiophen-H) und 2,85 ppm (1H, OH) als im Wesentlichen 4-(2,3-Dihydrothieno[3,4-b][1,4]dioxin-2-yl)-1-butanol identifiziert.

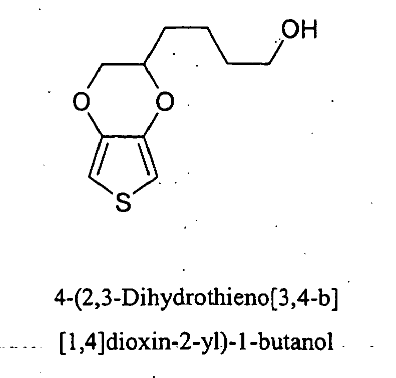

## Patentansprüche

1. Verbindungen der Formel I, 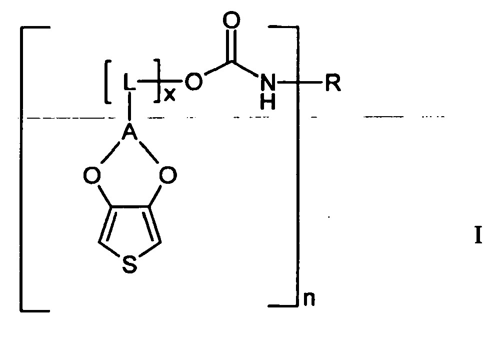 **dadurch gekennzeichnet, dass**
A für einen C₁-C₅-Alkylenrest steht, der an beliebiger Stelle gegebenenfalls über einen Linker L durch eine Urethangruppe substituiert ist und gegebenenfalls weitere Substituenten trägt,
L für eine Methylengruppe,
x für 0 oder eine ganze Zahl von 1 und größer steht,
n für eine ganze Zahl von 1 bis 4 steht und,
R für einen n-wertigen linearen oder verzweigten, gegebenenfalls substituierten, aliphatischen C₁-C₂₂-Rest, einen n-wertigen, gegebenenfalls substituierten, cycloaliphatischen C₃-C₁₂-Rest oder einen n-wertigen, gegebenenfalls substituierten, aromatischen C₆-C₁₄-Rest steht,
wobei für den Fall, dass n größer 1 ist, die Bedeutung von A und x unabhängig voneinander gleich oder verschieden sein kann.

2. Verbindungen der Formel I gemäß Anspruch 1, **dadurch gekennzeichnet, dass** x für 0 oder eine ganze Zahl von 1 bis 6 steht.

3. Verbindungen der Formel I gemäß Anspruch 1 oder 2, 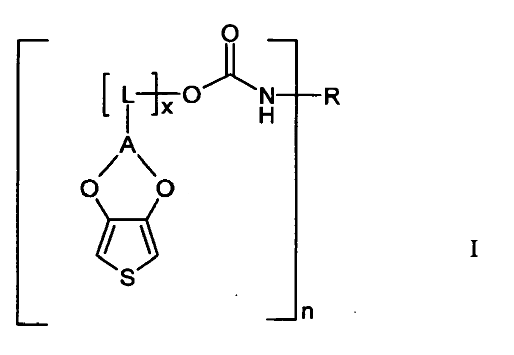 **dadurch gekennzeichnet, dass**
A für einen C₁-C₅-Alkylenrest steht, der an beliebiger Stelle gegebenenfalls über einen Linker L durch eine Urethangruppe substituiert ist und gegebenenfalls weitere Substituenten trägt,
L für eine Methylengruppe,
x für 0 oder 1 steht,
n für eine ganze Zahl von 1 bis 4 steht und,
R für einen n-wertigen linearen oder verzweigten, gegebenenfalls substituierten, aliphatischen C₁-C₂₂-Rest, einen n-wertigen, gegebenenfalls substituierten, cycloaliphatischen C₃-C₁₂-Rest oder einen n-wertigen, gegebenenfalls substituierten, aromatischen C₆-C₁₄-Rest steht,
wobei für den Fall, dass n größer 1 ist, die Bedeutung von A und x unabhängig voneinander gleich oder verschieden sein kann.

4. Verbindungen der Formel II gemäß wenigstens einem der Ansprüche 1 bis 3, 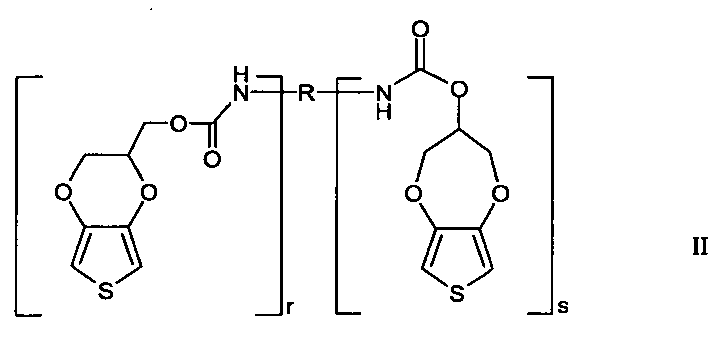 **dadurch gekennzeichnet, dass**
R für einen n-wertigen linearen oder verzweigten, gegebenenfalls substituierten, aliphatischen C₂-C₂₂-Rest, einen n-wertigen, gegebenenfalls substituierten, cycloaliphatischen C₅-C₁₂-Rest, einen n-wertigen, gegebenenfalls substituierten, aromatischen C₆-C₁₄-Rest steht,
r und s unabhängig voneinander für ganze Zahlen von 0 bis 4 stehen, mit der Maßgabe, dass r + s = n ist und
n für eine ganze Zahl von 1 bis 4 steht.

5. Verbindungen gemäß wenigstens einem der Ansprüche 1 bis 4, **dadurch gekennzeichnet, dass** R für einen linearen, gegebenenfalls substituierten C₆-C₁₂₋Alkylenrest oder einen gegebenenfalls substituierten C₆-C₁₀-Cycloalkylenrest steht, r und s unabhängig voneinander für ganze Zahlen von 0 bis 2 stehen und n = r + s = 2 ist.

6. Verbindungen gemäß wenigstens einem der Ansprüche 1 bis 5 der Formeln III und/oder IV, 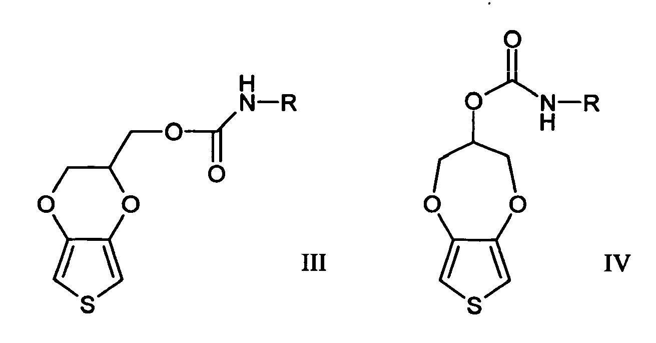 **dadurch gekennzeichnet, dass**
R für einen linearen oder verzweigten, gegebenenfalls substituierten C₁-C₂₂₋Alkylrest, einen gegebenenfalls substituierten C₃-C₁₂-Cycloalkylrest oder einen gegebenenfalls substituierten C₆-C₁₄-Arylrest steht.

7. Verbindungen gemäß wenigstens einem der Ansprüche 1 bis 6, **dadurch gekennzeichnet, dass** R für einen linearen C₁-C₁₂-Alkylrest oder einen C₆-C₁₀-Cycloalkylrest steht.

8. Verbindungen gemäß wenigstens einem der Ansprüche 1 bis 7, **dadurch gekennzeichnet, dass** R für einen linearen C₄-C₁₀-Alkylrest steht.

9. Verfahren zur Herstellung der Verbindungen der Formeln I bis IV, **dadurch gekennzeichnet, dass** Hydroxyverbindungen der Formel V, 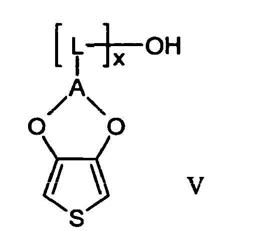 wobei
A, x und L die in den Ansprüchen 1 bis 8 genannte Bedeutung haben, mit Monoisocyanaten, Di- und/oder höherfunktionellen Isocyanaten
R(NCO)ₙ,
wobei
R und n die in den Ansprüchen 1 bis 8 genannte Bedeutung haben, umgesetzt werden.

10. Verfahren zur Herstellung von Oligo- und Poly(alkylendioxythiophen)en mit Urethangruppierungen enthaltenden Seitengruppen, **dadurch gekennzeichnet, dass** Verbindungen der Formeln I bis IV polymerisiert werden.

11. Oligo- und Poly(alkylendioxythiophen)e mit Urethangruppierungen enthaltenden Seitengruppen, **dadurch gekennzeichnet, dass** sie durch Polymerisation wenigstens einer Verbindung gemäß wenigstens einem der Ansprüche 1 bis 8 erhältlich sind.

12. Oligo- und Poly(alkylendioxythiophen)e mit Urethangruppierungen enthaltenden Seitengruppen gemäß Anspruch 11, **dadurch gekennzeichnet, dass** sie wiederkehrende Einheiten der Formeln VI a und/oder VI b enthalten 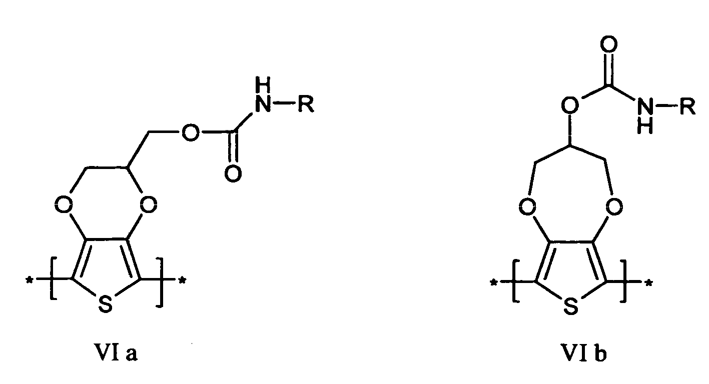 wobei
R die in wenigstens einem der Ansprüche 1 bis 8 genannte Bedeutung hat und die Zahl der wiederkehrenden Einheiten VI a und/oder VI b mindestens 2 ist.

13. Oligo- und Poly(alkylendioxythiophen)e mit Urethangruppierungen enthaltenden Seitengruppen gemäß wenigstens einem der Ansprüche 11 und 12, **dadurch gekennzeichnet, dass** die Zahl der Einheiten der Formel VI a und die Zahl der Einheiten der Formel VI b unabhängig voneinander jeweils 0 bis 1000 ist, mit der Maßgabe dass die Summe der Zahl der wiederkehrenden Einheiten der Formeln VI a und/oder VI b im Oligo- oder Poly(alkylendioxythiophen) mindestens 2 und höchstens 2000 ist.

14. Oligo- und Poly(alkylendioxythiophen)e mit Urethangruppierungen enthaltenden Seitengruppen gemäß wenigstens einem der Ansprüche 11 bis 13, **dadurch gekennzeichnet, dass** die Zahl der Einheiten der Formel VI a 1 bis 50 ist und unabhängig davon die Zahl der Einheiten der Formel VI b 0 bis 30 ist, mit der Maßgabe dass die Summe der Zahl der wiederkehrenden Einheiten der Formeln VI a und/oder VI b im Oligo- oder Poly(alkylendioxythiophen) mindestens 3 und höchstens 50 ist.

15. Oligo- und Poly(alkylendioxythiophen)e mit Urethangruppierungen enthaltenden Seitengruppen gemäß wenigstens einem der Ansprüche 11 bis 14, **dadurch gekennzeichnet, dass** sie positive Ladungen tragen.

16. Zwei- oder dreidimensional vernetzte neutrale oder kationische Oligo- und Poly(alkylendioxythiophen)e mit Urethangruppierungen enthaltenden Seitengruppen, **dadurch gekennzeichnet, dass** sie aus wenigstens einer der Verbindungen gemäß einem der Ansprüche 1 bis 8 durch oxidative Polymerisation erhältlich sind.

17. Verwendung der Oligo- und Poly(alkylendioxythiophen)e gemäß einem der Ansprüche 11 bis 16 als Bestandteil in elektrischen oder elektronischen Bauteilen.

## Claims

1. Compounds of the formula I, 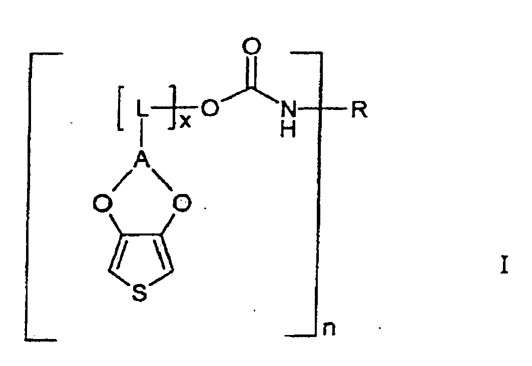 **characterised in that**
A stands for a C₁-C₅ alkylene radical which is substituted at any point optionally via a linker L by a urethane group and optionally supports further substituents,
L stands for a methylene group,
x stands for 0 or a whole number of 1 and greater,
n stands for a whole number from 1 to 4, and
R stands for an n-valent linear or branched, optionally substituted, aliphatic C₁-C₂₂ radical, an n-valent, optionally substituted, cycloaliphatic C₃-C₁₂ radical or an n-valent, optionally substituted, aromatic C₆-C₁₄ radical,
where for the case that n is greater than 1, the meaning of A and x independently of one another can be equal or different.

2. Compounds of the formula I according to claim 1, **characterised in that** x stands for 0 or a whole number from 1 to 6.

3. Compounds of the formula I according to claim 1 or 2, 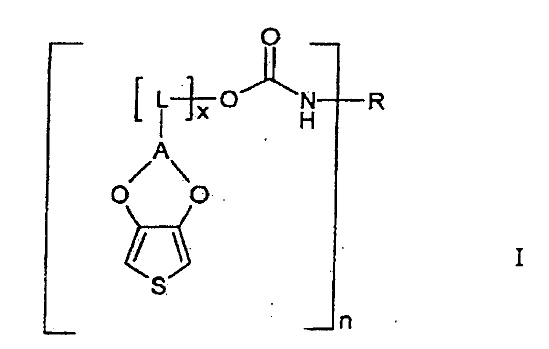 **characterised in that**
A stands for a C₁-C₅ alkylene radical which is substituted at any point optionally via a linker L by a urethane group and optionally supports further substituents,
L stands for a methylene group,
x stands for 0 or 1,
n stands for a whole number from 1 to 4, and
R stands for an n-valent linear or branched, optionally substituted, aliphatic C₁-C₂₂ radical, an n-valent, optionally substituted, cycloaliphatic C₃-C₁₂ radical or an n-valent, optionally substituted, aromatic C₆-C₁₄ radical,
where for the case that n is greater than 1, the meaning of A and x independently of one another can be equal or different.

4. Compounds of the formula II according to at least one of claims 1 to 3, 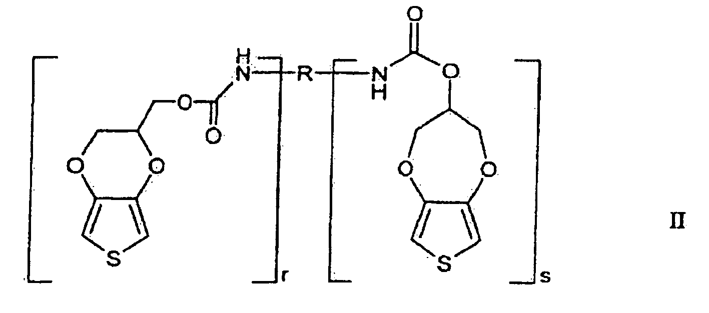 **characterised in that**
R stands for an n-valent linear or branched, optionally substituted, aliphatic C₂-C₂₂ radical, an n-valent, optionally substituted, cycloaliphatic C₅-C₁₂ radical, an n-valent, optionally substituted, aromatic C₆-C₁₄ radical,
r and s independently of one another stand for whole numbers from 0 to 4, with the proviso
that r + s = n and
n stands for a whole number from 1 to 4.

5. Compounds according to at least one of claims 1 to 4, **characterised in that** R stands for a linear, optionally substituted C₆-C₁₂ alkylene radical or an optionally substituted C₆-C₁₀ cycloalkylene radical, r and s independently of one another stand for whole numbers from 0 to 2 and n = r + s = 2.

6. Compounds according to at least one of claims 1 to 5 of the formulae III and/or IV, 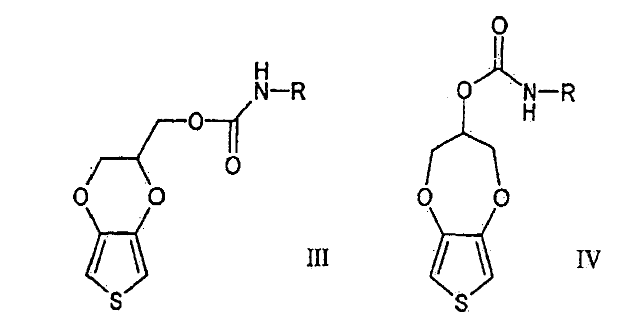 **characterised in that**
R stands for a linear or branched, optionally substituted C₁-C₂₂ alkyl radical, an optionally substituted C₃-C₁₂ cycloalkyl radical or an optionally substituted C₆-C₁₄ aryl radical.

7. Compounds according to at least one of claims 1 to 6, **characterised in that** R stands for a linear C₁-C₁₂ alkyl radical or a C₆-C₁₀ cycloalkyl radical.

8. Compounds according to at least one of claims 1 to 7, **characterised in that** R stands for a linear C₄-C₁₀ alkyl radical.

9. Process for preparing compounds of the formulae I to IV, **characterised in that** hydroxy compounds of the formula V, 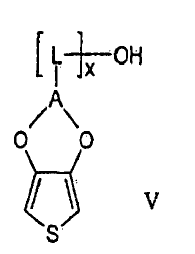 where
A, x and L have the meaning stated in claims 1 to 8, are reacted with monoisocyanates, di- and/or higher-functional isocyanates
R(NCO)ₙ,
where
R and n have the meaning stated in claims 1 to 8.

10. Process for preparing oligo- and poly(alkylenedioxythiophene)s with side groups containing urethane groups, **characterised in that** compounds of the formulae I to IV are polymerised.

11. Oligo- and poly(alkylenedioxythiophene)s with side groups containing urethane groups, **characterised in that** they are obtainable by polymerisation of at least one compound according to at least one of claims 1 to 8.

12. Oligo- and poly(alkylenedioxythiophene)s with side groups containing urethane groups according to claim 11, **characterised in that** they contain repeating units of the formulae VI a and/or VI b 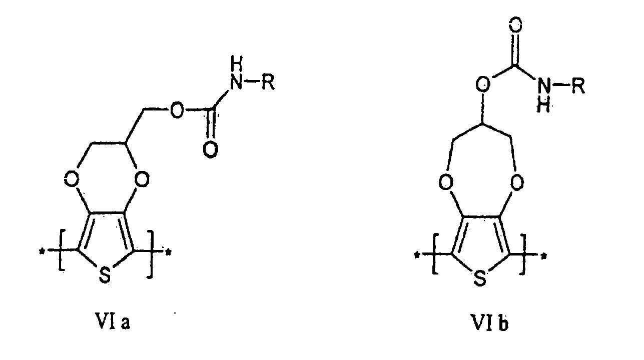 where
R has the meaning stated in at least one of claims 1 to 8 and the number of repeating units VI a and/or VI b is at least 2.

13. Oligo- and poly(alkylenedioxythiophene)s with side groups containing urethane groups according to at least one of claims 11 and 12, **characterised in that** the number of units of the formula VI a and the number of units of the formula VI b is each independently of one another 0 to 1000, with the proviso that the sum of the number of repeating units of the formulae VI a and/or VI b in the oligo- or poly(alkylenedioxythiophene) is at least 2 and at most 2000.

14. Oligo- and poly(alkylenedioxythiophene)s with side groups containing urethane groups according to at least one of claims 11 to 13, **characterised in that** the number of units of the formula VI a is 1 to 50 and independently thereof the number of units of the formula VI b is 1 to 30, with the proviso that the sum of the number of repeating units of the formulae VI a and/or VI b in the oligo- or poly(alkylenedioxythiophene) is at least 3 and at most 50.

15. Oligo- and poly(alkylenedioxythiophene)s with side groups containing urethane groups according to at least one of claims 11 to 14, **characterised in that** they carry positive charges.

16. Two- or three-dimensionally crosslinked neutral or cationic oligo- and poly(alkylenedioxythiophene)s with side groups containing urethane groups, **characterised in that** they are obtainable from at least one of the compounds according to one of claims 1 to 8 by oxidative polymerisation.

17. Use of the oligo- and poly(alkylenedioxythiophene)s according to one of claims 11 to 16 as constituent in electrical or electronic components.

## Revendications

1. Composés de la formule I, 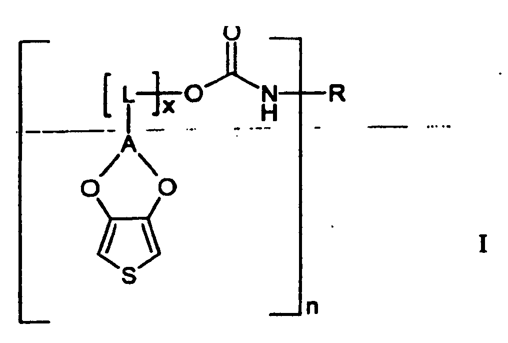 **caractérisés en ce que**
A représente un reste alkylène en C₁-C₅ qui est substitué en un endroit quelconque par un groupe uréthanne, le cas échéant via une jonction L, et porte le cas échéant des substituants supplémentaires,
L représente un groupe méthylène,
x représente 0 ou un nombre entier égal ou supérieur à 1,
n représente un nombre entier de 1 à 4 et,
R représente un reste aliphatique en C₁-C₂₂ linéaire ou ramifié de valence n, le cas échéant substitué, un reste cycloaliphatique en C₃-C₁₂ de valence n, le cas échéant substitué, ou un reste aromatique en C₆-C₁₄ de valence n, le cas échéant substitué,
dans le cas où n est supérieur à 1, la signification de A et x pouvant être indépendamment l'un de l'autre identique ou différente.

2. Composés de la formule I suivant la revendication 1, **caractérisés en ce que** x représente 0 ou un nombre entier de 1 à 6.

3. Composés de la formule I suivant la revendication 1 ou 2, 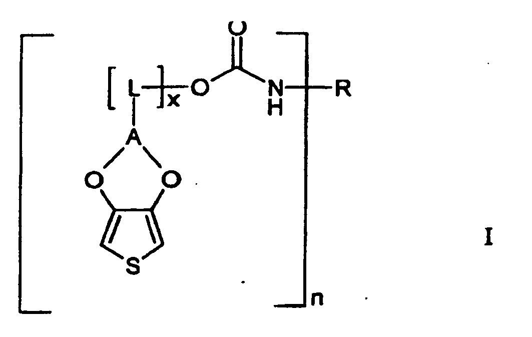 **caractérisés en ce que**
A représente un reste alkylène en C₁-C₅ qui est substitué en un endroit quelconque par un groupe uréthanne, le cas échéant via une jonction L, et porte le cas échéant des substituants supplémentaires,
L représente un groupe méthylène,
x représente 0 ou 1,
n représente un nombre entier de 1 à 4 et,
R représente un reste aliphatique en C₁-C₂₂ linéaire ou ramifié de valence n, le cas échéant substitué, un reste cycloaliphatique en C₃-C₁₂ de valence n, le cas échéant substitué, ou un reste aromatique en C₆-C₁₄ de valence n, le cas échéant substitué,
dans le cas où n est supérieur à 1, la signification de A et x pouvant être indépendamment l'un de l'autre identique ou différente.

4. Composés de la formule II suivant au moins l'une des revendications 1 à 3, 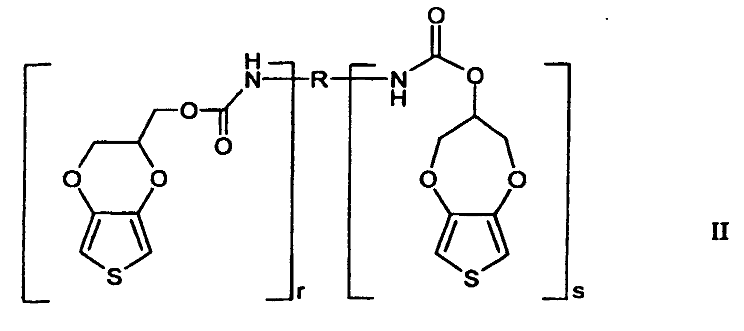 **caractérisés en ce que**
R représente un reste aliphatique en C₂-C₂₂ linéaire ou ramifié de valence n, le cas échéant substitué, un reste cycloaliphatique en C₅-C₁₂ de valence n, le cas échéant substitué, ou un reste aromatique en C₆-C₁₄ de valence n, le cas échéant substitué, r et s représentant indépendamment l'un de l'autre des nombres entiers de 0 à 4 avec la condition que r + s = n et
n représente un nombre entier de 1 à 4.

5. Composés suivant au moins l'une des revendications 1 à 4, **caractérisés en ce que** R représente un reste alkylène linéaire en C₆-C₁₂, le cas échéant substitué ou un reste cycloalkylène en C₆-C₁₀, le cas échéant substitué, r et s représentant indépendamment l'un de l'autre des nombres entiers de 0 à 2 et n = r + s = 2.

6. Composés suivant au moins l'une des revendications 1 à 5 des formules III et/ou IV, 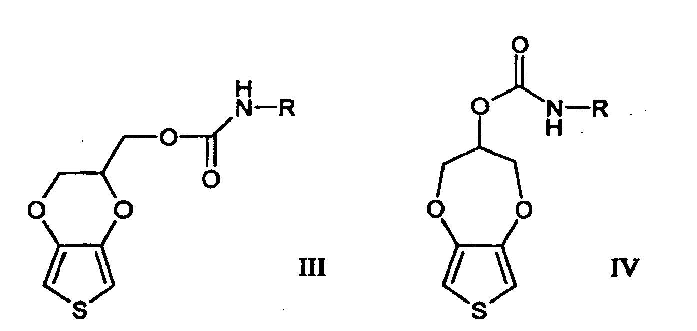 **caractérisés en ce que**
R représente un reste alkyle en C₁-C₂₂ linéaire ou ramifié, le cas échéant substitué, un reste cycloalkyle en C₃-C₁₂, le cas échéant substitué, ou un reste aryle en C₆-C₁₄, le cas échéant substitué.

7. Composés suivant au moins l'une des revendications 1 à 6, **caractérisés en ce que** R représente un reste alkyle linéaire en C₁-C₁₂ ou un reste cycloalkyle en C₆-C₁₀.

8. Composés suivant au moins l'une au moins des revendications 1 à 7, **caractérisés en ce que** R représente un reste alkyle linéaire en C₄-C₁₀.

9. Procédé de fabrication des composés des formules I à IV, **caractérisés en ce que** des composés hydroxylés de la formule V, 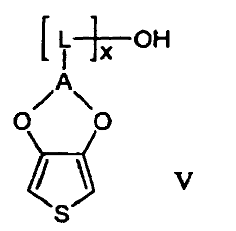 où
A, x et L ont la signification mentionnée dans les revendications 1 à 8, sont mis en réaction avec des monoisocyanates, des diisocyanates et/ou des isocyanates de fonctionnalité supérieure
R(NCO)ₙ,
R et n ayant la signification mentionnée dans les revendications 1 à 8.

10. Procédé de fabrication d'oligoalkylènedioxythiophènes et poly(alkylènedioxythiophène)s avec des groupes latéraux contenant des groupements uréthannes, **caractérisé en ce qu'**on polymérise des composés des formules I à IV.

11. Oligoalkylènedioxythiophènes et poly(alkylènedioxythiophène)s avec des groupes latéraux contenant des groupements uréthannes, **caractérisés en ce qu'**ils sont disponibles par polymérisation d'au moins un composé suivant au moins l'une des revendications 1 à 8.

12. Oligoalkylènedioxythiophènes et poly(alkylènedioxythiophène)s avec des groupes latéraux contenant des groupements uréthannes suivant la revendication 11, **caractérisés en ce qu'**ils contiennent des unités de répétition des formules VI a et/ou VI b 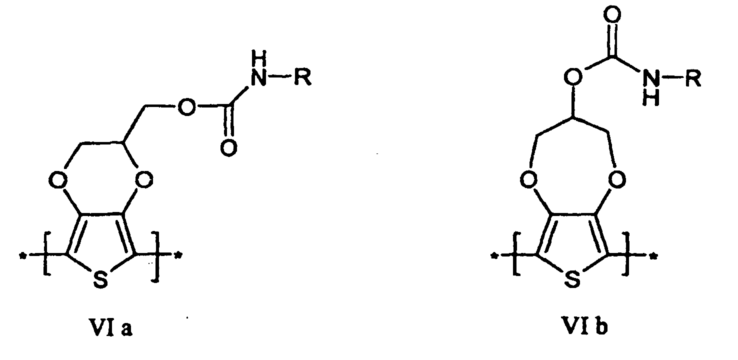 où
R a la signification mentionnée dans au moins l'une des revendications 1 à 8 et le nombre des unités de répétition VI a et/ou VI b est d'au moins 2.

13. Oligoalkylènedioxythiophènes et poly(alkylènedioxythiophène)s avec des groupes latéraux contenant des groupements uréthannes suivant au moins l'une des revendications 11 et 12, **caractérisés en ce que** le nombre des unités de la formule VI a et le nombre des unités de la formule VI b sont indépendamment l'un de l'autre respectivement de 0 à 1000, avec la condition que la somme des nombres des unités de répétition des formules VI a et/ou VI b dans les oligoalkylènedioxythiophènes ou poly(alkylènedioxythiophène)s est au moins 2 et au maximum 2000.

14. Oligoalkylènedioxythiophènes et poly(alkylènedioxythiophène)s avec des groupes latéraux contenant des groupements uréthannes suivant au moins l'une des revendications 11 à 13, **caractérisés en ce que** le nombre des unités de la formule VI a est de 1 à 50 et que le nombre des unités de la formule VI b est indépendamment de cela de 0 à 30, avec la condition que la somme des nombres des unités de répétition des formules VI a et/ou VI b dans les oligoalkylènedioxythiophènes ou poly(alkylènedioxythiophène)s est au moins 3 et au maximum 50.

15. Oligoalkylènedioxythiophènes et poly(alkylènedioxythiophène)s avec des groupes latéraux contenant des groupements uréthannes suivant au moins l'une des revendications 11 à 14, **caractérisés en ce qu'**ils portent des charges positives.

16. Oligoalkylènedioxythiophènes et poly(alkylènedioxythiophène)s neutres ou cationiques réticulés en deux ou en trois dimensions, avec des groupes latéraux contenant des groupements uréthannes, **caractérisés en ce qu'**ils sont disponibles par polymérisation oxydative à partir d'au moins un des composés suivant l'une des revendications 1 à 8.

17. Utilisation des oligoalkylènedioxythiophènes et poly(alkylènedioxythiophène)s suivant l'une des revendications 11 à 16 comme élément dans des composants électriques ou électroniques.
